(19) Europäisches Patentamt

European Patent Office

Office européen des brevets

(11) **EP 1 314 438 A1**

(12) **EUROPEAN PATENT APPLICATION**

(43) Date of publication:
28.05.2003 Bulletin 2003/22

(51) Int Cl.[7]: **A61K 45/06**, A61K 35/78,
A61P 35/00, A61P 17/06

(21) Application number: 01204495.4

(22) Date of filing: 23.11.2001

(84) Designated Contracting States:
**AT BE CH CY DE DK ES FI FR GB GR IE IT LI LU
MC NL PT SE TR**
Designated Extension States:
**AL LT LV MK RO SI**

(71) Applicant: **Nutricia N.V.
2700 MA Zoetermeer (NL)**

(72) Inventors:
• **Helvoort, Adrianus Lambertus Bertholdus
6708 SP Wageningen (NL)**

• **van Norren, Klaske
6871 LP Renkum (NL)**
• **Hageman, Robert Johan Joseph
6705 CT Wageningen (NL)**
• **Verwilligen, Wendy Antoinette
3404 CB Ijsselstein (NL)**
• **Lansink, Mirian
3524 BJ Utrecht (NL)**

(74) Representative: **van Westenbrugge, André et al
Nederlandsch Octrooibureau
P.O. Box 29720
2502 LS The Hague (NL)**

(54) **Anti-proliferative composition**

(57)    Non-estrogen-dependent hyperproliferation of cells in animals or humans can be prevented or treated by means of a pharmaceutical or nutritional composition containing a combination of

a) two or more inhibitors of the G1/S phase of the cell cycle; and
b) two or more inhibitors of the G2/M phase of the cell cycle; and
c) two or more inhibitors of protein tyrosine kinase activity.

Especially, component a) comprises two or more compounds selected from flavanolignans, carotenoids and isoflavone; component b) comprises two or more compounds selected from flavanolignans, hydroxylated stilbenes, isoflavones and apigenin; and component c) comprises two or more compounds selected from flavanolignans and isoflavones.

EP 1 314 438 A1

**Description**

*Field of the invention*

The present invention relates to the use of a composition comprising a combination of inhibitors of different phases of the cell cycle and inhibitors of protein tyrosine kinase activity for the prevention and treatment of hyperproliferation of cells. Furthermore, the invention concerns compositions with these anti-proliferative properties.

*Background of the invention*

Hyperproliferation of cells is in many cases a highly undesired process in man and animals, which can result in a variety of serious and sometimes fatal diseases. The most prominent members of these diseases are cancers. Cancer accounts for a significant portion of all deaths of men in the world. For instance in the United States about 1.2 million new cases of invasive cancer are diagnosed each year and about 500,000 people die annually of the disease. It is the second most deadly disease and it is expected to surpass heart disease early in the twenty-first century to top that nefarious list. Cancers may kill by the destructive invasion of normal organs through direct extension and spread to distant sites via the blood, lymph, or serosal surfaces.

Cancer comprises a class of diseases that can be characterised by the uncontrolled growth of aberrant cells. This uncontrolled growth of cells can be caused by hyperproliferation. In normal life mammalian tissue is constantly subjected to stressors (chemicals such as benzene or nitrosamines, mechanical damage, physical agents such as gamma and ultraviolet radiation and biologic agents such as the Epstein-Barr and hepatitis viruses), which contribute to carcinogenesis under certain circumstances by damaging or mutating cells or specific (essential) components therein. In the healthy body, several mechanisms are operational that are able to repair these abnormalities. When the cell cannot be repaired, it will die in a controlled way (apoptosis) and the tissue will replace the cell by a healthy one. When mutated or damaged cells are not recognised as undesirable, when they have become insensitive to the control of the environment or when they are able to divide rapidly (rapid cell proliferation), a tumour can be formed.

Several genes and factors are known to be involved in the formation and growth of tumours. In this respect mitogenic growth factors are well known. These growth factors can increase the activity of protein tyrosine kinase (PTK), which can phosphorylate several key proteins that regulate the cell cycle and therefore proliferation. Inhibition of PTK is a known target for cancer treatment policies, but treatment with synthetic PTK inhibitors results in severe negative side effects. Furthermore, several endogenous genes can be held responsible for uncontrolled cell growth. One group of these genes is called oncogenes. Proto-oncogenes, the oncogene precursors, act as biochemical switches in cellular command and control processes, specifically relaying signals from the outside of the cell to the nucleus. The progressive and controlled transfer of extracellular signals is bypassed when one of the relay members is rendered constitutively activated by an oncogenic mutation. This results in uncontrolled cell division.

In addition to the increase of positive growth signals, a decrease in cell loss as well as an increase in cellular proliferation can be the cause of uncontrolled cell growth. Whereas proto-oncogenes are identified by a gain of function after mutational damage, another class of cancer genes, the so-called tumour-suppressor genes, contribute to uncontrolled cell growth by a loss in function after mutational damage. Normally, these genes can prevent division of damaged DNA by binding to specific genes and modulating their expression. This results in a regulation of the expression of certain proteins that play a role as checkpoints during the cell cycle. When the tumour-suppressor genes are mutated this regulation is decreased or even completely absent, leading to uncontrolled proliferation of cells.

Cell proliferation occurs in a cycle of events. An ordered set of actions ensures that one cell will divide into two daughter cells with identical genetic material as the mother cell. These can each further divide into two new daughter cells. Non-dividing cells are per definition in a so-called G0 phase. A gap phase (G1) occurs before the cell enters the so-called "synthesis" (S) phase in which DNA is replicated. After the synthesis phase a second gap phase (G2) can be recognised before the cell enters the mitosis (M) stage in which the nucleus (and chromosomes) and cytoplasm separate (cytokinesis). In the M stage four phases can be recognised.

Thus, chromosome replication and segregation are confined to discrete parts of the cell cycle, whereas the third essential component of cell reproduction - growth - occurs continuously in all phases, G1, S, G2 and M. During G1 and G2, cells can respond to proliferative and anti-proliferative signals such as growth factors and cytokines, that determine whether cell division ought to proceed.

At present, tumours are often intensively examined before treatment in order to determine the optimal treatment strategy. The main therapeutic modalities for cancer comprise surgery, radiotherapy, chemotherapy and biologic therapy. Surgery is the oldest modality but alone often inadequate because of metastasis of cancer cells. Radiotherapy is most useful for localised tumours that cannot be resected or for tumours that tend to spread to predictable contiguous sites. Chemotherapy is used for a systemic treatment for any cancer. It often consists of a combination of drugs. These combinations of drugs need to be administered in order to create the highest probability of success. The composition of these cocktails can also be subject to change when during therapy the characteristics of the tumour cells change. Finally, biologic therapies of cancer are under development for the initial stages. These include, in addition to bone marrow transplantation, treatment with compounds such as lymphokines, monoclonal antibodies or agents such as retinoic acid causing tumour cells to undergo differentiation to "harmless" cells.

Many forms of cancer are very difficult to treat using current therapies. This applies certainly for non-estrogen-dependent cancers like prostate cancer and colon cancer. Despite applications of available therapies, the patients suffering from these cancers often have a bad prognosis for survival in the longer term. In many instances, this is due to the fact that either the active drug is unable to penetrate the tumour in sufficient amounts, or tumour cells have become resistant to the drug used. Furthermore, cancer therapies, especially chemotherapy, cause serious undesirable side effects, which hinder proper functioning of the patients for a shorter or longer period of time. These potential side effects include sickness, tiredness, weight loss, malfunctioning, boldness, infertility, etc. Cancer therapies are also known to cause damage to the mucosal lining of for example mouth, throat and intestine. In addition, patients often feel a loss in hunger sensation. This leads to bad eating habits and even malnutrition, which further impart the recovery process and prognosis.

Although the success rate of the main cancer therapies has increased in recent years, a lot of research is done to reveal the causes of cancer and to develop better therapies. In the treatment of tumours it is important that the inhibition of the proliferation of tumour cells occurs in a selective way. Proliferation of other rapidly dividing cells and metabolically highly active cells such as fibroblasts gut, epithelial cells, bone marrow and cells that have a strong endocrine function should ideally not be hindered. During tumour growth new blood vessels must be created (angiogenesis) in order to increase the capacity for supply of nutrients and oxygen to the tumour and for the removal of metabolites from the tumour. It is therefore important that therapeutic preparations inhibit proliferation of the tumour cells as well as the cells that are involved in tumour-induced angiogenesis.

US 5,912,265 (Bombardelli et al.) discloses the anti-proliferative activity of flavanolignans selected from the group silymarin, silybin, silidianin, silicristin and dehydrosilybin or mixtures or extracts thereof on tumours that are estrogen-dependent. Growth of these tumours increases by higher systemic estrogen levels. Examples of estrogen-dependent cancers are ovary, breast, cervix and uterus cancers. The flavanolignans or components thereof are shown to possess antagonistic activity on type II estrogen receptors or receptors beta. No reference is made to the activity of these flavanolignans or components thereof against proliferation of tumour cells from non-estrogen-dependent cancers such as tumours in prostate, colon, lung, skin, bladder, etc. The flavanolignans can be combined with anti-tumour agents cisplatin or adriamycin, and with lipids.

WO 00/07607 (Kosbab) discloses multi-component compositions for use in the prevention or treatment of cancer and osteoporosis, especially female cancers, including hormone-dependent cancers. The compositions comprise a combination of antioxidants, neovascular regulators, collagen factors, minerals, vitamins, arginine and other amino acids and many more components. The antioxidants consist of a wide variety of components, including vitamins C and E, zinc, potassium and bioflavonoids from plants. The bioflavonoids include catechins, tannins from various plant extracts, resveratrol, silymarin, curcumin, quercetin, lutein, β-carotenes, and glutathione being mentioned among many other antioxidants. The neovascular regulators include chondroitin sulphate, protamine sulphate, isoflavones (e. g. genistein, daidzein), *Gymnema sylvestre* and others. The collagen factors include glucosamine, chondroitin sulphate, manganese and certain amino acids. As an example, formula III contains leucoanthocyanidins, ginkgo biloba, vitamins C, E and A, limonene, carotenoids (possibly lycopene), tea polyphenols, genistein, chondroitin sulphate, zinc, calcium and magnesium, arginine and ω-3 fatty acids; formula VI contains the components of formula III and in addition protamine sulphate, vitamin D3, branched amino acids, quercitin, saw palmetto, vitamin B complex, potassium, selenium, thioctic acid, allicin, silymarin, curcumin, niacinamide, linoleic acid, and some more. Amounts to be administered range from a few micrograms or milligrams to several hundreds or even thousands of milligrams per day for most components, but for the specific formulae as described above, no amounts are given for any of the components.

A disadvantage of the compositions of WO 00/07607 is that they become bulky and expensive. Furthermore, no relation to the anti-proliferative properties of the compositions is suggested, nor to the synergy that can be obtained by administering various components that interfere with several stages in cell proliferation, nor to the specificity of the activity of the preparation for tumour cells and the resulting absence of significant undesired side effects. The components of the compositions are clearly included for a wide range of other effects (see Table 2 in WO 00/07607). A further disadvantage of the compositions of WO 00/07607 is that they comprise several components such as arginine and certain glycosaminoglycans possessing an undesirable promoting effect on ischaemia and PMA-induced angiogenesis (see Murohara *et al.* (1998) J. Clin. Invest. 101, 2567-2578), which may promote tumour growth. WO 00/07606 is silent on the desirability of combining the multi-component compositions with carbohydrates, fats and proteins, and on the nature of these energy providers.

WO 01/26668 (Schroeder et al.) describes compositions with anti-prostate cancer activity containing lycopene, selenium compounds and isoflavonoids (genistein, daidzein, etc). The further components phytosterols, catechins, β-carotenes, lutein and tocopherols are said to enhance the effectivity of the composition. The preferred ratio of isoflavonoids to lycopene is 12:1. A split administration (isoflavonoids and catechins in several dosages per day, and the other components only once a day) is preferred.

*Summary of the invention*

Surprisingly, it has now been found that compositions which comprise several components that act on several stages of the cell cycle and that are able to inhibit PTK-and growth factor-induced cell proliferation, have a stronger anti-proliferative activity than prior art compositions on a much wider range of mutated and non-mutated cells. Such compositions are therefore not only useful in the prevention and treatment of a broad range of cancers, especially non-estogen-dependent cancers, but also in the prevention and management or treatment of other diseases characterised by hyperproliferation of certain cells, such as psoriasis, overgrowth of non-malignant cells and some chronic inflammatory diseases at certain stages, such as stages where hyperproliferation of mesenchyme cells occurs during inflammatory bowel disease (IBD), in particular ulcerative colitis. It was found that, in order to have full anti-proliferative activity, a composition should interfere with all proliferation mechanisms described above, i.e. first gap (G1), synthesis (S), second gap (G2) and mitosis (M), referred ot herein as the three phases G1/S, G2/S and M. An effective composition should always act on these three mechanisms and each mechanism should be affected by at least two different agents, to reduce the risk of resistance to the treatment.

*Description of the invention*

It was found according to the invention that a combination of components comprising:

a) two or more inhibitors of the G1/S phase of the cell cycle; and
b) two or more inhibitors of the G2/M phase of the cell cycle; and
c) two or more inhibitors of protein tyrosine kinase activity,
can be used for the preparation of a composition for the prevention and treatment of hyperproliferation of cells in animals or humans.

Component a) comprises two or more inhibitors of the G1/S phase of the cell cycle. These inhibitors can be either of synthetic or natural origin, whereby compounds of natural origin are preferred. Preferably, the inhibitors of the G1/S phase of the cell cycle comprise compounds selected from flavanolignans, lycopene, daidzein and daidzin or equol or functional analogues thereof, such as glycosides, sulphates, esters, and lactone, but also other inhibitors of the G1/S phase of the cell cycle which are known in the art can be used, as well as other natural inhibitors of the G1/S phase of the cell cycle, or their synthetic equivalents. Functional analogues according to the invention are compounds that are present as the same compounds in the gut after consumption.

Component a) can comprise flavanolignans to be used in amounts of 10-1400 mg, preferably 20-500 mg per daily dose. Furthermore, it can comprise carotenoids in an amount of 0.1-100 mg, preferably 5-20 mg per daily dose and/or daidzein in an amount of 5-1000 mg, preferably 30-200 mg per daily dose.

The flavanolignans can comprise silymarin, or one or more of its constituents and analogues such as silybin, silydianin, silycristin, dehydrosilybin and mixtures thereof. Preferably, silymarin is used as a flavanolignan. Flavanolignans and in particular silymarin are known to be effective in the treatment of several diseases such as Amanita Mushroom poisening, hepatitis and cirrhosis. They can be synthesised but preferably they are purified from natural sources such as the milk thistle (*Silybum marianum*). Flavanolignans are found in the highest concentrations in extracts of the fruit portion of the milk thistle but extracts of other parts of that plant such as the leaves or seeds are suitable as sources to obtain flavanolignans. Methods for isolation and purification of the flavanolignans are well known in the art. Other lignans, such as enterolactone (2,3-bis(3-hydroxybenzyl)butyrolactone, are also effective as inhibitors of the G1/S cell cycle phase.

Carotenoids are well known compounds that play a role in plants, presumably in the management of photon energy. Carotenoids and retinoids according to the invention are preferably obtained from natural sources. They preferably comprise lycopene. Lycopene occurs in ripe fruits, such as pink grapefruit 30-40 ppm, watermelon (*Citrullus lanatus*) 40-900 ppm, *Aglaonema commutatum, Arbarus unedo,* guava, paprika (*Capsicum annuum*), apricot (*Prunus armeniaca*), peach (*Prunus persica*), *Momordica charantia,* carrot (*Daurus carota*) 80-140 ppm, papaya (*Carica papaya*) and especially tomatoes (*Lycopersicon esculentum*) 1-35 ppm in fruit, 50-80 ppm in tomato paste, 80-120 ppm in ketchup. It may also be derived from other fruits in which lycopene biosynthesis is expressed. Methods for isolation and purification of carotenoids and in particular lycopene are well known in the art.

Another preferred inhibitor of the G1/S phase of the cell cycle comprises daidzein and its functional analogues such as for instance daidzin. Daidzein and its functional analogues can be synthesised but they are preferably obtained from natural sources such as extracts of soy beans or red clover. Methods for isolation and purification of daidzein and its functional analogues are known in the art.

Component b) comprises two or more inhibitors of the G2/M phase of the cell cycle. These inhibitors can be either of synthetic or natural origin, whereby compounds of natural origin are preferred. Preferably, the inhibitors of the G2/M phase of the cell cycle comprise compounds selected from flavanolignans, hydroxylated stilbenes, genistin and functional analogues thereof such as genestein, etherified, esterified or glycosylated forms, but also other inhibitors of the G2/M phase of the cell cycle which are known in the art can be used, such as apigenin and quercetin.

Component b) can comprise flavanolignans to be used in amounts of 10-1400 mg, preferably 20-500 mg per daily dose, and/or hydroxylated stilbenes in an amount of 0.1-100 mg, preferably 5-20 mg per daily dose and/or genistein in an amount of 5-1000 mg, preferably 30-200 mg per daily dose.

Hydroxylated stilbenes according to the invention preferably comprise resveratrol (3,5,4'-trihydroxystilbene) but other hydroxylated stilbenes and functional analogues known in the art such as the trans isomer of 3,5-dihydroxystilbene (pinosylvine), 3,3'-dihydroxystilbene, 3,4'-dihydroxystilbene and 3,5,3'-trihydroxystilbene and glycosylated forms thereof can also be used. The hydroxylated stilbenes according to the invention, in particular resveratrol, can either be synthesized or isolated from natural sources. These sources which are preferred include inter alia aqueous or ethanolic extracts of *Polygonum spp.* and *Vitis spp.*

Another preferred inhibitor of the G2/M phase of the cell cycle comprises genistein and its functional analogues such as for instance genistin. Genistein and its functional analogues can be synthesized but they are preferably obtained from natural sources such as extracts of soy beans. Methods for isolation and purification of genistein and its functional analogues are known in the art.

Component c) comprises two or more inhibitors of protein tyrosine kinase activity. These inhibitors can be either of synthetic or natural origin, whereby compounds of natural origin are preferred. Preferably, the inhibitors of protein tyrosine kinase activity comprise compounds selected from flavanolignans, isoflavones and functional analogues thereof such as silymarin and genistein, but also other inhibitors of protein tyrosine kinase activity which are known in the art can be used, such as fatty acid derivatives of amino acids as disclosed in US 5,216,023, apigenin, and hydroxylated stilbenes such as resveratrol.

Component c) can comprise flavanolignans to be used in amounts of 5-1000 mg, preferably 5-50 mg per daily dose and/or isoflavones in an amount of 5-1000 mg, preferably 40-200 mg per daily dose.

Isoflavones are a group of compounds called phytoestrogens, or also called plant estrogens. They are mainly found in legumes and plant seeds and in particular in soy beans and red clover. The primary isoflavones in soy beans are genistein and daidzein. These forms of isoflavones are called aglycone forms since they do not contain carbohydrate groups. The glycone form which contains carbohydrate groups however is the main form of isoflavones in plants. Examples of glycones are genistin and daidzin. Isoflavones to be used in component c) preferably comprise genistein. As mentioned above for genistein, isoflavones can be synthesised, but they are preferably obtained from natural sources such as extracts of soy beans and red clover. Methods for isolation and purification of isoflavones and their functional analogues are known in the art.

In particular, these inhibitors of the G1/S phase and G2/M phase of proliferation and of tyrosine kinase are active in a concentration as defined in table 1 below. The inhibitory concentration of the active components is defined by the activity of these components in the following *in vitro* assays.

*Cell cycle analysis to determine G1/S and G2/M inhibition.*

LNCaP cells are grown to subconfluency and incubated for 24 hours with various concentrations of the bioactive component or with a vehicle control. Cells are harvested and fixed with 70% ethanol in PBS. Cell pellet is then resuspended in 0.5 ml PBS containing propidium iodide (50 μg/ml) and DNase-free RNase (100 μg/ml). Cell cycle analysis is performed by using a FACS analysis. An effective G1/S arrest is defined by a > 1.5 fold increase in the area under the G1-curve. Similarly a G2/M arrest is defined by a > 2 fold increase in the area under the G2-curve at a given concentration.

*Protein tyrosine kinase inhibition assay.*

After 36 hours serum starvation, 70-80% confluent Du-145 cell are incubated for 2 hours extracts with various concentrations of the bioactive component or with a vehicle control. After this preincubation the cells are treated for 15 minutes at 37°C with either TNFα (50ng/ml) or PBS. After cell lysis in ice-cold buffer (10mM Tris, pH7.4, 150mM NaCl, 1% Triton X-100, 1mM EDTA, 1mM EGTA, 0.2 mM sodium vanadate, 0.2 mM PMSF, 0.5% NP-40, 0.2 units/ml aprotinin) erbB1 is immunoprecipitated using anti-EGFR, which is followed by Western blotting and probing with anti-phosphotyrosine. $IC_{50}$ values for tyrosine kinase inhibition are based on the reduction of the anti-phosphotyrosine signal.

Table 1:

| Effective concentrations of proliferation inhibitors | | | |
|---|---|---|---|
| Effective concentration | G1/S cell cycle arrest | G2/M cell cycle arrest | Inhibition tyrosine kinase activity |
| Required | < 150 μM | < 150 μM | < 200 μM |
| Preferably | < 80 μM | < 80 μM | <100 μM |
| More preferably | < 50 μM | <50 μM | < 70 μM |
| Most preferably | < 25 μM | < 25 μM | < 50 μM |

In a preferred embodiment according to the invention, component a) contains a flavanolignan and a carotenoid or daidzein, component b) contains a flavanolignan and a hydroxylated stilbene or genistein and component c) contains a flavanolignan and genistein, and these components are used to prepare a composition for the prevention and treatment of hyperproliferation of cells in animals or humans. An especially preferred composition comprises a flavanolignan, particularly silymarin, a soy extract and lycopene.

In a preferred use according to the invention the sum of the total daily dose of components a), b) and c) is between 0.5 and 35 mg per kg body weight in animals and humans, or between about 35 and about 2500 mg per human per day, preferably between 70 and 700 mg per human per day.

In a preferred embodiment of the use according to the invention the composition comprises a lipid fraction, in an amount of e.g. 2-800 parts by weight, especially 8-200 parts by weight per part of inhibitors a), b) and c) taken together. This lipid fraction is present in the composition to increase the bioavailability of the above-mentioned components. The lipid fraction can comprise triglyceride oils, partial glycerides, surfactants and cosurfactants. The lipid fraction preferably comprises those components to support the auto-emulsifying process as occurs in the gastrointestinal tract during absorption of the bioactive compounds. Thus, the lipid faction preferably comprises triglyceride oils and phospholipids. Examples of triglyceride oils are olive oil, sunflower oil, corn oil and MCT (medium chain triglyeride) oil, but also lipids containing higher amounts of saturated fatty acids such as coconut or palm kernel lipids. Examples of phospholipids include phosphatidyl choline, phosphatidyl ethanolamine and phosphatidyl serine, but also lysophospholipids. Extracts from soybeans, rapeseed and eggs are the preferred sources of phospholipids. The use of lysophospholipids is beneficial for the manufacture of emulsions for topical administration. The surfactant activity of the phospholipids is especially achieved when more than 30% of the phospholipids is phosphatidylcholine. Glycerol can be included in the product of the invention to support the auto-emulsifying process and to improve the taste of the product.

Furthermore, it is preferred that the ratio of the weight per daily dose of the sum of the components a), b) and c) to the weight per daily dose of the lipid fraction is between 1:300 and 2:1. For supplements to be used in combination with regular meals, a ration of between 1:6 and 2:1, especially about 1:1, is preferred; for supplements to be used independently, a ratio of a) +b) +c) to lipids between 1:30 and 1:6 is preferred. In another embodiment of the use according to the invention the lipid can be chemically attached to a synthetically produced compounds, for example by synthesising phospholipid esters of isoflavones.

In a preferred embodiment of the use according to the invention the composition further can comprise one or more beneficial other herbal extracts or components thereof such as extracts that comprise baicalein or baicalin or licochalcone, vitamins such as vitamine C, E, A and D, trace elements such as zinc, selenium, the latter especially as selenium-enriched garlic or yeast, minerals, antioxidants and macroingredients (fats, carbohydrates and/or proteins). These compounds can be included according to the methods known in the art. Furthermore, it is preferred to include a fibre source that can generate significant amounts of butyrate in the colon in the composition according to the invention. An example of such a fibre source is wheat bran, inulin and fractions thereof, and resistant starch. The amount of this fibre source should preferably exceed 1 g per daily dose.

In the use according to the invention the compositions are preferably dietetic and/or pharmaceutical compositions. They can be administered as a complete meal or as a supplement and are very palatable in terms of flavour, taste and consistancy of the product and comprise little or no undesirable side effects. The compositions according to the invention can be administered, preferably orally, to an animal or human in liquid forms or dry forms, such as for instance as drinks, emulsions, jellies, puddings, ice creams, soups, sauces, bars, aqueous or oily suspensions, syrups or elixirs or in dry forms such as for instance as tablets, troches, lozenges, dispersible powders or granules, hard capsules or soft gelatin capsules, or as powder or premix concentrate to be reconstituted to liquid forms. A preferred embodiment from organoleptic point of view is a bar-type product, in particular a bar having a chocolate taste. The products can also be suitable for topical use, such as for the prevention and treatment of psoriasis and warts. A suitable application form is a spray, e.g. as a sprayable emulsion.

According to the invention the compositions can be used for the prevention and treatment of diseases associated with hyperproliferation in animals and humans. Examples of diseases characterised by among others hyperproliferation of certain cells are inter alia psoriasis, overgrowth of non-malignant cells such as warts, benign prostate hyperplasia and adenomatous polyps, and some chronic inflammatory diseases at certain stages such as inflammatory bowel disease (IBD), in particular ulcerative colitis.

In particular, the compositions according to the invention are claimed to be effective against uncontrolled proliferation of a broad range of mutated tumour cells at various stages of development. More in particular the compositions according to the invention are claimed to be especially effective against uncontrolled proliferation of tumour cells whose growth is not promoted by estrogens. Examples of such diseases are inter alia prostate, lung, bladder, skin, bloodcell (Hodgkin, leukaemia), pancreas, liver, kidney, mouth, larynx, oesophagus, stomach, spleen, rectum, small intestine and colon.

Next to decreasing the rate of tumour growth and degree of metastasis formation, the compositions according to the invention can be used to maintain low proliferation rates of tumour cells, e.g. in periods before surgery when tumour sizes are small. The compositions can be used alone or in combination with current therapies suitable for the prevention or treatment of diseases associated with hyperproliferation, and in particular cancers.

The present invention further relates to a composition that does not promote angiogenesis. Agents that are known to promote angiogenesis, such as arginine and glycosaminoglycans, should not be present or only at a low level. In two animal models it was found that arginine stimulates angiogenesis (see Murohara *et al.* (1998) J. Clin. Invest. 101, 2567-2578), which increases the capacity for supply of nutrients and oxygen to the tumour and for the removal of metabolites from the tumour. Therefore, the composition of the invention preferably comprises:

a) two or more inhibitors of the G1/S phase of the cell cycle; and
b) two or more inhibitors of the G2/M phase of the cell cycle; and
c) two or more inhibitors of protein tyrosine kinase activity,

whereby the ratio of the weight of the sum of components a), b) and c) to the weight of L-arginine is larger than 1:1, preferably larger than 4:1, and the amount of L-arginine leads to an L-arginine dose of < 10 mg per day. Preferably, the composition according to the invention comprises no arginine. Also, it is preferred that the composition according to the invention contains no more than low amounts of glycosamine sulphates and glycosaminoglycans, such as chondroitin sulphate and glucosamine sulphate; in particular the levels of such compounds are less than 0.1, more preferably less than 0.02 times the total amount of components a), b) and c), or in absolute amounts, less than 50

mg, or even less than 10 mg per day.

**Experimental part**

*Cell proliferation as a function of the administration of components of the invention*

***Materials and methods***

*Test components*

Isoflavones (genistein, daidzein, equol) were purchased from Indofine Chemical Company, Inc. (New Jersey, USA). Silymarin, a mixture of flavonolignans from the fruit of silybum marianum, was purchased Sigma-Aldrich Chemie (Zwijndrecht, Netherlands). Lycopene was provided by Roche Diagnostics (Mannheim, Germany). Stock solutions were prepared as follows: Genistein and silymarin were dissolved in ethanol. Daidzein and equol were dissolved in dimethyl sulfoxide (DMSO). Lycopene was dissolved in 0.02M HCl

*Cell culture*

The PC3 cell line (American Type Culture Collection, Mannassas, USA), a highly resistant prostate cancer cell line originating from a metastasis lesion of bone, was maintained as monolayer cultures in RPMI medium (Gibco, Life technologies BV, Breda, Netherlands) supplemented with 5% FCS (Gibco). The cells were grown mycoplasm free in plastic tissue culture flasks (Falcon, Micronic, Lelystad, Netherlands). Cultures were kept in a 5% CO2 humidified atmosphere, at a temperature of 37°C. Media were replaced every 2 or 3 days, and cells were subcultured at weekly intervals using a 0.05% trypsin and 0.01% EDTA.

*Exposure to test components.*

The PC3 cells were seeded into flat-bottomed 96-well tissue culture treated plates (Falcon, Micronic, Lelystad, Netherlands) at a density of 16000 cells per well. After 24h incubation, vehicle controls, test components or combinations of the test components were added to the medium (20 µl added to 200 µl medium in well). The components were tested in combination to determine additional or synergistic effects. PC3 cells were exposed for 4 days.

*Proliferation assays*

Cell proliferation was quantified by measuring the incorporation of 5-bromo-2'-deoxyuridine (BrdU) during DNA synthesis and by cleavage of tertazolium salt (WST-1) in viable cells by mitochondrial dehydrogenases. The colorimetric BrdU (immunoassay) and colorimetric WST-1 assay were performed in accordance with the manufacturer's instructions (Roche Diagnostics Mannheim, Germany). Briefly, BrdU-assay: After the exposure period, the cells were treated with BrdU-labeling solution for 3 hours. After labeling, cells were washed with PBS, fixed for 30 minutes and washed again with PBS. After 90 minutes incubation with the anti- BrdU-POD working solution, the cells were washed 3 times. After addition of the substrate, the reaction was stopped with $H_2SO_4$. The absorbance was measured at 450 nm with a reference wavelength at 650nm. WST-1 assay: After the exposure period, the cells were washed with PBS and treated with WST-1 diluted in medium (1:10). After 3 hours incubation, the absorbance was measured at 450nm with a reference at 595 nm.

*Classification of the inhibitory effects:*

Synergistic effects of the combinations of different components were those, which inhibits the proliferation significantly greater than the values of combinations of the components alone. Additive effects were dose in which the proliferation was not significantly greater than the value of the combinations of the components alone but the inhibition of the proliferation was significant greater than the values of the components alone.

***Results***

*Combination effects of genistein, equol, lycopene and silymarin on proliferation of the human prostate cancer cell line PC3.*

Figure 1 shows the effects of genistein, equol, lycopene and silymarin either alone or in combination on the proliferation of prostate cancer cell line PC3. Genistein and equol alone resulted in an inhibition of proliferation of human prostate cancer cell line PC3 of 20.2±4.6%, 30.1±4.4% (mean ± s.e.) respectively. Silymarin and 1ycopene alone do not inhibited growth of the PC3 cells significant (9.5±4%, 7.3±2.7%, respectively). Combinations of silymarin and either genistein, equol or lycopene additionally inhibited growth by 52.3±3.7%, 64.4±1.1% and 46.0±3.4% respectively. Genistein with equol or lycopene additionally inhibited growth by 73.1±0.7% and 91.5±4.1% and lycopene together with equol additionally inhibited the growth by 91.6±3.3%. Combinations of three or more components additionally inhibited growth by more than 99%.

**Example 1**

Composition for the treatment of prostate cancer

The following components were mixed:

| Component | Amount per day (mg) |
|---|---|
| Soy isoflavones | 100.0 |
| Lycopene | 15.0 |
| Silymarin | 160.0 |
| Antioxidants | |
| Vitamin C | 225.0 |
| Vitamin E | 75.0 |
| Carotenoids | 3.0 |
| Flavonoids | 19.0 |
| Ubiquinol | 4.0 |
| Selenium | 0.128 |
| Zinc | 18.0 |
| Copper | 2.7 |
| Manganese | 5.0 |
| Riboflavin | 2.5 |
| Vitamin B6 | 3.3 |
| Vitamin B12 | 0.0033 |
| Folate | 0.4 |
| N-Acetyl-cysteine | 500 |

## Example 2

Composition for the prevention of colorectal cancer

The following components were mixed:

| Component | Amount per day (mg) |
|---|---|
| Soy isoflavones | 25.0 |
| Lycopene | 2.0 |
| Silymarin | 50.0 |
| Fibre mix | 15000 |
| Calcium | 1500 |
| Probiotics | |
| Lb. rhamnosus + Lb. acidophilus | 2-4 $10^{10}$ CFU |
| Antioxidants | |
| Vitamin C | 150.0 |
| Vitamin E | 37.5 |
| Carotenoids | 1.5 |
| Flavonoids | 10.0 |
| Ubiquinol | 2.0 |
| Selenium | 0.085 |
| Zinc | 9.0 |
| Copper | 1.4 |
| Manganese | 2.5 |
| Riboflavin | 1.3 |
| Vitamin B6 | 1.3 |
| Vitamin B12 | 1.6 |
| Folate | 0.4 |
| Green tea extract | 300.0 |
| Curcumin | 60.0 |

**Example 3**

Conventional chocolate bar of 45 g. Ingredients: sugar syrup, milk powder, chocolate mass, cocoa butter, soy lecithin and flavours, in which 1 g sugar syrup is replaced by glycerol and 1.5 g of the chocolate mass is replaced by 50 mg milk thistle extract, 20 mg apigenin and 20 mg lycopene.

**Example 4**

Sauce for a hot meal containing per 100 g dry mass:

| Milk thistle extract | 50 mg (providing 42 mg silymarin) |
|---|---|
| Tomato concentrate | 20 g (providing 2 mg lycopene) |
| Carrot powder | 2 g (providing 7 mg lycopene) |
| Full fat soy flower | 40 g |
| (provides 8.2 g of lipids, of which 0.8 g phospholipids) | |
| Garlic | 1 g (can be replaced by yeast) |
| Modified starch | 5 g |
| Flavours (thyme, rosemary) | 1 g |
| Vegetable concentrate | 20 g |
| Sodium chloride | 10 g |

**Example 5**

Composition for the treatment of benign prostate hyperplasia (BPH) and prevention of prostate cancer
The following components were mixed:

| Component | Amount per day (mg) |
|---|---|
| Soy isoflavones | 30 |
| providing genistein | 10 |
| Lycopene | 5 |
| Silybum marianum | 50 |
| *Serence repens* (saw palmetto) | 320 |
| lipophilic extract | 272 |
| Selenium | 0.10 |
| Zinc | 15 |
| Copper | 2 |
| *Prunus africana* extract | 50 |
| providing sterols (12%) | 6 |
| Soybean oil | 500 |
| Soy lecithin | 200 |

Other ingredients: capsule shell: gelatine, glycerin, natural source colours: sulphite ammonia caramel, titanium dioxide; anti-caking agents: silicon dioxide.

**Example 6**

Creme for topical application on the skin. The following premeixes were prepared:

| A: | Mineral oi1 | 10 % |
|---|---|---|
| | Squalane | 5 % |
| | Dimethicone | 3% |
| | PEG-30 dipolyhydroxystearate | 3 % |
| B: | Glycerol | 6% |

(continued)

|   |   |   |
|---|---|---|
|   | Magnesium sulphate heptahydrate | 0.7 % |
|   | Water | up to 100% |
| C: | Preservatives | 0.5% |
|   | Perfumes | 0.5% |
|   | Active components of the invention | 2 % |

The active components in C are the following:

|   |   |
|---|---|
| Resveratrol (from grape seeds) | 20 mg |
| Lycopene (from tomato) | 40 mg |
| Apigenin | 20 mg |
| Enterolactone | 10 mg |

The creme is prepared by heating A and B to 75°C, adding B to A whilst stirring, homogenising for 1 minute, allowing to cool to 40°C whilst stirring, adding C, and allowing to cool to ambient temperature whilst stirring.

**Claims**

1. Use of a combination of

    a) two or more inhibitors of the G1/S phase of the cell cycle; and
    b) two or more inhibitors of the G2/M phase of the cell cycle; and
    c) two or more inhibitors of protein tyrosine kinase activity, for the preparation of a composition for the prevention and treatment of non-estrogen-dependent hyperproliferation of cells in animals or humans.

2. Use according to claim 1, wherein component a) comprises two or more compounds selected from flavanolignans, carotenoids, isoflavones and functional analogues thereof.

3. Use according to claim 2, wherein component a) contains flavanolignans in an amount of 10-1400 mg, preferably 20-500 mg per daily dose and/or carotenoids in an amount of 0.1-100 mg, preferably 5-20 mg per daily dose and/or daidzein in an amount of 5-1000 mg, preferably 30-200 mg per daily dose.

4. Use according to any of claims 1-3, wherein component b) comprises two or more compounds selected from flavanolignans, hydroxylated stilbenes, isoflavones, apigenin and functional analogues thereof.

5. Use according to claim 4, wherein component b) contains flavanolignans in an amount of 10-1400 mg, preferably 20-500 mg per daily dose and/or hydroxylated stilbenes in an amount of 0.1-100 mg, preferably 5-20 mg per daily dose and/or genistin in an amount of 5-1000 mg, preferably 30-200 mg per daily dose.

6. Use according to any of claims 1-5, wherein component c) comprises two or more compounds selected from flavanolignans, isoflavones and functional analogues thereof.

7. Use according to claim 6, wherein component c) contains flavanolignans in an amount of 5-1000 mg, preferably 5-50 mg per daily dose and/or genistein in an amount of 5-1000 mg, preferably 40-200 mg per daily dose.

8. Use according to any one of claims 2-7, wherein said flavanolignans comprise one or more compounds selected from silymarin and constituents and functional analogues thereof.

9. Use according to any of claims 1-8, which comprises silymarin, a soy extract and lycopene.

10. Use according to any of claims 1-9, wherein the sum of the total daily dose of components a), b) and c) is between 0.5 and 35 mg per kg body weight in animals and humans.

11. Use according to any of claims 1-10, wherein the composition comprises a lipid fraction.

**12.** Use according to claim 11, wherein the lipid fraction comprises phospholipids.

**13.** Use according to claims 11 or 12, wherein the ratio of the weight per daily dose of the sum of the components a), b) and c) to the weight per daily dose of the lipid fraction is between 1:300 and 2:1, preferably between 1:30 and 1:6.

**14.** Use according to any of the claims 1-13, wherein the composition comprises one or more herbal extracts or components thereof.

**15.** Use according to any of the claims 1-14, wherein the composition further comprises one or more compounds selected from vitamins, trace elements, minerals, antioxidants and macroingredients.

**16.** Use according to any of claims 1-15 for the prevention and treatment of non-estrogen associated cancers in animals or humans, especially prostate or colorectal cancers, or for the treatment of psoriasis.

**17.** A composition comprising:

a) two or more inhibitors of the G1/S phase of the cell cycle; and
b) two or more inhibitors of the G2/M phase of the cell cycle; and
c) two or more inhibitors of protein tyrosine kinase activity,

**characterised in that**, the ratio of the weight of the sum of components a), b) and c) to the weight of L-arginine is > 1:1.

**18.** A composition according to claims 19, which contains silymarin, genistein and/or daidzein, and lycopene in relative amounts of 1 : 0.2-4 : 0.02-0.4.

**European Patent Office**

## PARTIAL EUROPEAN SEARCH REPORT

which under Rule 45 of the European Patent Convention shall be considered, for the purposes of subsequent proceedings, as the European search report

**Application Number**

EP 01 20 4495

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | ZHOU JIN-RONG ET AL: "Soybean phytochemicals inhibit the growth of transplantable human prostate carcinoma and tumor angiogenesis in mice." JOURNAL OF NUTRITION, vol. 129, no. 9, 1999, pages 1628-1635, XP002192993 ISSN: 0022-3166 * abstract; figure 1D * | 1,2,4,6, 17 | A61K45/06 A61K35/78 A61P35/00 A61P17/06 |
| X | WO 99 48386 A (STUECKLER FRANZ) 30 September 1999 (1999-09-30) * claim 7 * * page 19, line 20 - line 31 * | 17 | |
| X | WO 00 07607 A (KOSBAB JOHN V) 17 February 2000 (2000-02-17) | 17 | |
| Y | *Formulation 7B* * tables 1,2 * | 14,15 | |
| | -/-- | | |

|  |
|---|
| **TECHNICAL FIELDS SEARCHED (Int.Cl.7)** |
| A61K A61P |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 26 March 2002 | Böhmerova, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**EP 1 314 438 A1**

| European Patent Office | INCOMPLETE SEARCH SHEET C | Application Number EP 01 20 4495 |
|---|---|---|

Claim(s) searched completely:
   9, 18

Claim(s) searched incompletely:
   1-8, 10-17

Reason for the limitation of the search:

Present claims 1 and 17 relate to the use of compounds defined by reference to a desirable characteristic or property, namely to ability thereof to inhibit:
- G1/S phase of the cell cycle,
- G2/M phase of the cellcylce and
- tyrosine kinase activity
in the preparation of mediacment for the treatment of estrogen-independent hyperproliferation or cells
or a pharmaceutical composition comprising combinations of such compounds, respectively.

In general, the definiton of compounds in terms of functional parameters makes a complete search impossible. In this regards, it is not always disclosed in the searched prior art documents whether candidate compound(s) would fulfill the requirements of such functional parameters or not.
Moreover, present claims 1 to 8 and 10 to 17 relate to an extremely large number of possible combinations of compounds. In fact, the claims contain so many possible permutations that a lack of clarity and conciseness within the meaning of Article 84 EPC arises to such an extent as to render a meaningful search of the claims impossible. Consequently, the search has been carried out for those parts of the application which do appear to be clear and concise, namely to the combinations claimed in dependent claims 9 and 18 and disclosed in the examples.

14

**European Patent Office**

## PARTIAL EUROPEAN SEARCH REPORT

Application Number

EP 01 20 4495

| Category | DOCUMENTS CONSIDERED TO BE RELEVANT | | CLASSIFICATION OF THE APPLICATION (Int.Cl.7) |
|---|---|---|---|
| | Citation of document with indication, where appropriate, of relevant passages | Relevant to claim | |
| Y | HSIEH TZE-CHEN ET AL: "Differential effects on growth, cell cycle arrest, and induction of apoptosis by resveratrol in human prostate cancer cell lines." EXPERIMENTAL CELL RESEARCH, vol. 249, no. 1, 25 May 1999 (1999-05-25), pages 109-115, XP002192994 ISSN: 0014-4827 * abstract * * page 113, right-hand column, line 26 - line 31; figure 1; table 1 * | 1-8, 10-17 | |
| Y | SANTELL ROSS C ET AL: "Genistein inhibits growth of estrogen-independent human breast cancer cells in culture but not in athymic mice." JOURNAL OF NUTRITION, vol. 130, no. 7, July 2000 (2000-07), pages 1665-1669, XP002192995 ISSN: 0022-3166 * abstract; figure 1; table 1 * | 1-8, 10-17 | **TECHNICAL FIELDS SEARCHED** (Int.Cl.7) |
| Y | EL-ZARRUK A A ET AL: "The anti-proliferative effects of tyrosine kinase inhibitors towards tamoxifen-sensitive and tamoxifen-resistant human breast cancer cell lines in relation to the expression of epidermal growth factor receptors (EGF-R) and the inhibition of EGF-R tyrosine kinase." CANCER LETTERS, vol. 142, no. 2, 3 August 1999 (1999-08-03), pages 185-193, XP002192996 ISSN: 0304-3835 * abstract; figures 1,4 * | 1-8, 10-17 | |

-/--

EPO FORM 1503 03.82 (P04C10)

European Patent Office

# PARTIAL EUROPEAN SEARCH REPORT

**Application Number**

EP 01 20 4495

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| Y | BHATIA NEEHAR ET AL: "Detrimental effect of cancer preventive phytochemicals silymarin, genistein and epigallocatechin 3-gallate on epigenetic events in human prostate carcinoma DU145 cells." PROSTATE, vol. 46, no. 2, 1 February 2001 (2001-02-01), pages 98-107, XP002192997 ISSN: 0270-4137 * abstract; figure 7 * | 1-8, 10-17 | |
| Y | EP 0 600 544 A (MAKHTESHIM CHEM WORKS LTD) 8 June 1994 (1994-06-08) * claim 3 * * page 4, line 45 - line 47; example 3 * | 1-8, 10-17 | **TECHNICAL FIELDS SEARCHED** (Int.Cl.7) |
| Y | GUPTA SANJAY ET AL: "Selective growth-inhibitory, cell-cycle deregulatory and apoptotic response of apigenin in normal versus human prostate carcinoma cells." BIOCHEMICAL AND BIOPHYSICAL RESEARCH COMMUNICATIONS, vol. 287, no. 4, 5 October 2001 (2001-10-05), pages 914-920, XP002192998 ISSN: 0006-291X * page 919, left-hand column, line 37 - line 57; figure 4 * | 1-8, 10-17 | |
| Y | EP 0 659 402 A (INDENA SPA) 28 June 1995 (1995-06-28) * claim 6 * | 11-13 | |

EPO FORM 1503 03.82 (P04C10)

# EP 1 314 438 A1

## ANNEX TO THE EUROPEAN SEARCH REPORT
## ON EUROPEAN PATENT APPLICATION NO.

EP 01 20 4495

This annex lists the patent family members relating to the patent documents cited in the above-mentioned European search report.
The members are as contained in the European Patent Office EDP file on
The European Patent Office is in no way liable for these particulars which are merely given for the purpose of information.

26-03-2002

| Patent document cited in search report | | Publication date | Patent family member(s) | | Publication date |
|---|---|---|---|---|---|
| WO 9948386 | A | 30-09-1999 | AT | 407821 B | 25-06-2001 |
| | | | AT | 52598 A | 15-11-2000 |
| | | | WO | 9948386 A1 | 30-09-1999 |
| | | | AU | 2911399 A | 18-10-1999 |
| | | | CA | 2325437 A1 | 30-09-1999 |
| | | | EP | 1065946 A1 | 10-01-2001 |
| WO 0007607 | A | 17-02-2000 | AU | 5334899 A | 28-02-2000 |
| | | | EP | 1100517 A1 | 23-05-2001 |
| | | | WO | 0007607 A1 | 17-02-2000 |
| EP 0600544 | A | 08-06-1994 | IL | 103920 A | 26-07-2000 |
| | | | AU | 679467 B2 | 03-07-1997 |
| | | | AU | 5201493 A | 09-06-1994 |
| | | | CA | 2109750 A1 | 31-05-1994 |
| | | | CN | 1095589 A | 30-11-1994 |
| | | | EP | 0600544 A1 | 08-06-1994 |
| | | | JP | 6227970 A | 16-08-1994 |
| | | | US | 5827900 A | 27-10-1998 |
| | | | ZA | 9308791 A | 03-10-1994 |
| EP 0659402 | A | 28-06-1995 | IT | 1265312 B1 | 31-10-1996 |
| | | | AT | 214264 T | 15-03-2002 |
| | | | AU | 677048 B2 | 10-04-1997 |
| | | | AU | 6313294 A | 13-07-1995 |
| | | | CA | 2123739 A1 | 22-06-1995 |
| | | | CN | 1111506 A | 15-11-1995 |
| | | | DE | 659402 T1 | 10-10-1996 |
| | | | EP | 0659402 A2 | 28-06-1995 |
| | | | ES | 2081781 T1 | 16-03-1996 |
| | | | FI | 942452 A | 22-06-1995 |
| | | | GR | 96300007 T1 | 29-02-1996 |
| | | | JP | 7196534 A | 01-08-1995 |
| | | | US | 5648377 A | 15-07-1997 |

EPO FORM P0459

For more details about this annex : see Official Journal of the European Patent Office, No. 12/82

17